(19) Europäisches Patentamt

European Patent Office

Office européen des brevets

(11) **EP 0 943 316 B1**

(12) **EUROPÄISCHE PATENTSCHRIFT**

(45) Veröffentlichungstag und Bekanntmachung des Hinweises auf die Patenterteilung:
**15.03.2006 Patentblatt 2006/11**

(51) Int Cl.:
*A61K 8/67* (2006.01)      *A61K 8/33* (2006.01)
*A61K 8/35* (2006.01)      *A61K 8/365* (2006.01)
*A61K 8/46* (2006.01)      *A61K 8/44* (2006.01)
*A61K 8/66* (2006.01)      *A61Q 5/08* (2006.01)

(21) Anmeldenummer: **98119753.6**

(22) Anmeldetag: **22.10.1998**

(54) **Mittel zur Färbung und Entfärbung von Fasern**

Means for coloring and decoloring of hair

Agent pour colorer et decolorer des cheveux

(84) Benannte Vertragsstaaten:
**DE ES FR GB IT**

(30) Priorität: **12.03.1998 DE 19810688**

(43) Veröffentlichungstag der Anmeldung:
**22.09.1999 Patentblatt 1999/38**

(73) Patentinhaber: **Wella Aktiengesellschaft**
**64274 Darmstadt (DE)**

(72) Erfinder:
• **Le Cruer, Dominique**
**1723 Marly (CH)**
• **Dousse, Christel**
**1733 Treyvaux (CH)**
• **Kunz, Manuela, Dr.**
**1723 Marly (CH)**

(56) Entgegenhaltungen:
**DE-A- 4 216 667      DE-U- 29 810 215**
**FR-A- 2 657 781**

**Beschreibung**

Gegenstand der vorliegenden Erfindung ist ein Mittel zur Entfärbung von Fasern, ein Verfahren zur Entfärbung von Fasern unter Verwendung dieses Mittels sowie ein Mehrkomponenten-Kit zur Färbung und späteren Entfärbung von Fasern, insbesondere von menschlichen Haaren, der sowohl das Mittel zur Erzeugung einer Färbung auf der Faser als auch das erfindungsgemäße Mittel zur Entfärbung der Fasern enthält.

Oxidationsfärbemittel eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von bis zu 100 % oder zum "Hellerfärben" verwendeten Oxidationsfärbemittel in der Regel als sogenannte oxidative Farben bezeichnet werden.

Direktziehende Farbstoffe, insbesondere Nitrofarbstoffe, sind in nicht-oxidativen Färbemitteln (sogenannten Tönungsmitteln) weit verbreitet. Sie können aufgrund ihrer geringen Größe in das Haar eindringen und es - zumindestens in den äußeren Bereichen - direkt anfärben. Derartige Tönungen sind sehr haarschonend und überstehen in der Regel mehrere Haarwäschen.

Direktziehende Farbstoffe, insbesondere Nitrofarbstoffe, werden ebenfalls häufig in oxidativen Färbemitteln zur Erzeugung bestimmter Nuancen beziehungsweise zur Intensivierung der Farbe eingesetzt.

Es ist bekannt, daß oxidativ im Haar erzeugte farbige Polymere im allgemeinen sehr haltbar gegen äußere Einflüsse wie Wasser, Shampoo oder Licht sind. Je nach Färbetechnik sind sie so fest verankert, daß sie im allgemeinen bis zum nächsten Haarschnitt im Haar verbleiben. Ist eine Entfernung der Färbung gewünscht, müssen relativ agressive Chemikalien, wie Formaldehyd-sulfoxylate, Wasserstoffperoxid oder Wasserstoffperoxid-Additionsprodukte eingesetzt werden. Eine weitgehende Entfärbung ist so zwar möglich, ist aber gesundheitsschädlich oder mit Haarschädigungen verbunden.

Eine teilweise Entfärbung von nicht-oxidativen Tönungen ist in der Regel bereits durch mehrmaliges Haarewaschen möglich, eine gezielte und vollständige sofortige Entfernung der Haarfarbe ist auf diesem Wege jedoch nicht möglich.

Soll eine besondere Haarfarbe nur für einen kurzen Zeitraum getragen werden, ist daher sowohl bei oxidativen als auch bei nicht-oxidativen Färbungen die Entfernung der Haarfarbe unter milden und schonenden Bedingungen ein bisher ungelöstes Problem.

Erfindungsgemäß wird diese Aufgabe durch den Einsatz einer Kombination a) mindestens eines Reduktons, beispielsweise Ascorbinsäure, und/oder eines Thiols und/oder eines Sulfits und b) mindestens einer $\alpha$-Oxocarbonsäure oder deren physiologisch verträgliches Salz gelöst.

Der Einsatz von Ascorbinsäure in Haarpflegemitteln oder Haarfärbemitteln ist an sich bekannt. In der EP-PS 0 401 454 wird zum Beispiel vorgeschlagen, Reste von Wasserstoffperoxid, die nach einer oxidativen Behandlung im menschlichen Haar zurückbleiben, mit einer wäßrigen Lösung von Ascorbinsäure zu entfernen. Hierfür geeignet sind Ascorbinsäure enthaltende Brausetabletten, die unmittelbar vor der Anwendung in Wasser aufgelöst werden, das dann zur Haarspülung eingesetzt wird.

Weiterhin wird Ascorbinsäure in der DE-OS 1 444 216 in einem flüssigen Haarfärbemittel eingesetzt, um das sonst instabile flüssige Mittel haltbar zu machen. Auch das Oxidationshaarfärbemittel gemäß der DE-OS 3 642 097 enthält Ascorbinsäure als Stabilisator. Umso überraschender ist es, daß Ascorbinsäure vorteilhaft auch zur reduzierenden Entfernung von Oxidationsfarben aus Fasern, beispielsweise menschlichen Haaren, verwendet werden kann.

FR-A-2657781 offenbart Entfärbungsmitteln für Haare enthaltend Ascorbinsäure, Cystein oder Sulfit.

Gegenstand der vorliegenden Erfindung ist ein Mehrkomponenten-Kit zur Färbung und späteren Entfärbung von Fasern, insbesondere von Haaren, - welcher dadurch gekennzeichnet ist, daß er als Komponente (I) Mittel zur oxidativen oder nicht-oxidativen Färbung von Fasern, insbesondere menschlichen Haaren, und als Komponente (II) Mittel zur reduzierenden Entfernung der Färbung mit einem Gehalt an a) mindestens einem Redukton und/oder einem Thiol und/oder einem Sulfit und b) mindestens eine $\alpha$-Oxocarbonsäure oder deren physiologisch verträgliches Salz enthält.

Aus der DE-OS 42 16 667 ist es bekannt, $\alpha$-Ketodicarbonsäuren (beispielsweise $\alpha$-Ketoglutarsäure) oder deren physiologisch verträgliche Salze zur Beseitigung von Wasserstoffperoxidresten aus dem Haar zu verwenden. Weiterhin ist es aus der JP-OS 63-101 307 bekannt, als Zellaktivator zur Haarwuchsförderung beispielsweise eine $\alpha$-Ketoglutarsäure zu verwenden.

Die in dem erfindungsgemäßen Mehrkomponenten-Kit enthaltenen Mittel zur Erzeugung einer oxidativen Färbung (Komponente (I)) bestehen in der Regel aus einer Mischung von zwei Komponenten, nämlich einer Farbträgermasse, welche die als Entwicklersubstanz und Kupplersubstanz bezeichneten Farbstoffvorstufen und gegebenenfalls nicht-oxidative Farbstoffe enthält, und einem Oxidationsmittel, welches unmittelbar vor der Anwendung zwecks Bildung des Oxidationsfarbstoffes zugesetzt wird, während die in dem erfindungsgemäßen Mehrkomponenten-Kit enthaltenen Mittel zur Erzeugung einer nicht-oxidativen Färbung (Komponente (I)) in der Regel in Form eines Einkomponentenpräparates vorliegen.

Der erfindungsgemäße Mehrkomponenten-Kit enthält im Falle der oxidativen Färbung in der Farbträgermasse als Entwicklersubstanz mindestens eine zur Bildung von Oxidationsfarbstoffen geeignete Farbstoffvorstufe. Besonders geeignet hierfür sind 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylaminoanilin, 4-Dimethylamino-anilin, 4-Diethylamino-anüin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)-benzol, 1,3-Bis[(4 aminophenyl)(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylaminophenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1 H)-pyrimidon, 4,5-Diarnino-1-(2-hydroxyethyl)-1 H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol sowie 2-Amino-phenol, 2-Amino-6-methyl-phenol, 2-Amino-5-methyl-phenol und/oder deren Salze.

Außerdem enthält die Farbträgermasse im Falle der oxidativen Färbung mindestens eine zur Bildung einer Oxidationsfarbe geeignete Kupplersubstanz. Hierfür können aromatische m-Diamine, m-Aminophenole, Polyphenole oder Naphthole eingesetzt werden. Besonders geeignet sind N-(3-Dimethylamino-phenyl)-hamstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1-(2-Aminoethoxy)-2,4-diamino-benzol, 2-Amino-1 -(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxybenzol, 5-Methyl-2-(1-methylethyl)-phenol, 3-[(2-Hydroxyethyl)amino]-anilin, 3-[(2-Aminoethyl)amino]-anilin, 1,3-Di (2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion und/oder deren Salze.

Die Entwickrersubstanzen und Kupplersubstanzen sind in der Farbträgermasse jeweils in einer Menge von etwa 0,01 bis 10 Gewichtsprozent, vorzugsweise 0,1 bis 5 Gewichtsprozent, enthalten.

Weiterhin kann diese, Oxidationsfarbstoffe enthaltende, Farbträgermasse gegebenenfalls zusätzlich nicht-oxidative Farbstoffe (nachfolgend "direktziehende Farbstoffe" genannt), wie zum Beispiel 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4.-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)amino]-4-[ethyl-(2 -hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)-amino)-5-dimethylamino-benzoesäure (HC Blue No.13), 1-Amino-4-[(2-hydroxyethyl)amino]-2-nitrobenzol (HC Red No. 7), 2-Amino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxyethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6),1-Ghlor-2,4-bis[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,1 0-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500, Disperse Blue No. 7, Solvent Blue No. 69), 9-(Dimethylamino)-benzo[a]phenoxazin-7-ium-chlorid (Cl51175; Basic Blue No. 6), Di[4-(diethylamino)phenyl][4-(ethylamino)naphthyl]carbenium-chlorid (Cl42595; Basic Blue No. 7), 3,7-Di(dimethylamino)phenothiazin-5-ium-chlorid (Cl52015; Basic Blue No. 9), Di[4-(dimethylamino)phenyl][4-(phenylamino)naphthyl]carbenium-chlorid (Cl44045; Basic Blue No. 26), 2-[(4-(Ethyl(2-hydroxyethyl)amino)phenyl)azo]-6-methoxy-3-methyl-benzothiazolium-methylsulfat (Cl11154; Basic Blue No. 41), 8-Amino-2-brom-5-hydroxy-4-imino-6-[(3-(trimethylammonio)phenyl)amino]-1 (4H)-naphthalinon-chlorid (Cl56059; Basic Blue No. 99), Bis[4-(dimethylamino)phenyl][4-(methylamino)phenyl]carbenium-chlorid (Cl42535; Basic Violet No. 1), Tris[4-(dimethylamino)phenyl]carbenium-chlorid (Cl42555; Basic Violet No. 3), 2-[3,6-(Diethylamino)dibenzopyranium-9-yl]-benzoesäure-chlorid (Cl45170; Basic Violet No. 10), Di(4-aminophenyl)(4-amino-3-methylphenyl)carbenium-chlorid (Cl42510; Basic Violet No. 14), 1,3-Bis[(2,4-diamino-5-methylphenyl)azo]-3-methylbenzol (Cl21010; Basic Brown No. 4), 1-[(4-Aminophenyl)azo]-7-(trimethylammonio)-2-naphthol-chlorid (Cl12250; Basic Brown No. 16), 1-[(4-Amino-2-nitrophenyl)azoJ-7-(trimethylammonio)-2-naphthol-chlorid (Basic Brown No. 17), 1-[(4-Amino-3-nitrophenyl)azo]-7-(trimethylammonio)-2-naphthol-chlorid (Cl12251; Basic Brown No. 17), 3,7-Diamino-2,8-dimethyl-5-phenyl-phenazinium-chlorid (Cl50240; Basic Red No. 2), 1,4-Dimethyl-5-[(4-(dimethylamino)phenyl)azo]-1,2,4-triazolium-chlorid (Cl11055; Basic Red No. 22), 2-Hydroxy-1-[(2-methoxyphenyl)azo]-7-(trimethylammonio)-naphthaün-chlorid (Cl12245; Basic Red No. 76), 2-[2-((2,4-Dimettioxy-phenyl)amino)ethenyl]-1,3,3-trimethyl-3H-indol-1-ium-chlorid (Cl48055; Basic Yellow No. 11), 3-Methyl-1-phenyl-4-[(3-(trimethylammonio)phenyl)-azo]-pyrazol-5-on-chlorid (Cl12719; Basic Yellow No. 57), Bis[4-(diethylamino)phenyl]phenylcarbenium-hydrogensulfat (1:1) (Cl42040, Basic Green No. 1), 1-[Di(2-hydroxyethyl)amino]-3-methyl-4-[(4-nitrophenyl)azo]-benzol (Cl11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl10316; Acid Yellow No. 1; Food Yellow No. 1), 2-(Indan-1,3-dion-2-yl)chinolin-x,x-sulfonsäure (Gemisch aus Mono- und Disulfonsäure) (Cl47005;D&C Yellow No. 10; Food Yellow No. 13; Acid Yellow No. 3), 5-Hydroxy-1-(4-sulfophenyl)-4-[(4-sulfophenyl)azo]pyrazol-3-carbonsäure-trinatriumsalz (Cl19140; Food Yellow No. 4; Acid Yellow No. 23), 9-(2-Carboxyphenyl)-6-hydroxy-3H-xanthen-3-on (Cl45350; Acid Yellow No. 73; D&C Yellow No. 8), 5-[(2,4-Dinitrophenyl)amino]-2-phenylamino-benzolsulfonsäure-natriumsalz (Cl10385; Acid Orange No. 3), 4-[(2,4-Dihydroxyphenyl)azo]-benzolsulfonsäure-mononatriumsalz (Cl14270; Acid Orange No. 6), 4-[(2-Hydroxynaphth-1-yl)azo]-benzolsulfonsäure-natriumsalz (Cl15510; Acid Orange No. 7), 4-[(2,4-Dihydroxy-3-[(2,4-dimethylphenyl)azo]-phenyl)azo]-benzolsulfonsäure-natriumsalz (Cl20170; Acid Orange No. 24), 4-Hydroxy-3-[(4-sulfonaphth-1-yl)azo]-1-naphthalin-sulfonsäure-dinatriumsalz (Cl14720; Acid Red No. 14), 6-Hydroxy-5-[(4-sulfonaphth-1-yl)azo]-2,4-naphthalin-disulfonsäure-trinatriumsalz (Cl16255; Ponceau 4R; Acid Red No. 18), 3-Hydroxy-4-[(4-sulfonaphth-1-yl)azo]-2,7-naphthalin-disulfonsäure-trinatriumsalz (Cl16185; Acid Red No. 27), 8-Amino-1-hydroxy-2-(phenylazo)-3,6-naphthalin-disulfonsäure-dinatriumsalz (Cl17200; Acid Red No. 33), 5-(Acetylamino)-4-hydroxy-3-[(2-methylphenyl)azo]-2,7-naphthalin-disulfonsäure-dinatriumsalz (Cl18065; Acid Red No. 35), 2-(3-Hydroxy-2,4,5,7-tetraiod-dibenzopyran-6-on-9-yl)-benzoesäure-dinatriumsalz (Cl45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (Cl45100; Acid Red No. 52), 8-[(4-(Phenylazo)phenyl)azo]-7-naphthol-1,3-disulfonsäure-dinatriumsalz (C127290; Acid Red No. 73), 2',4',5',7'-Tetrabrom-3',6'-dihydroxyspiro[isobenzofuran-1(3H),9'-[9H]xanthen]-3-on-dinatriumsalz (C145380; Acid Red No. 87), 2',4',5',7'-Tetrabrom-4,5,6,7-tetrachlor-3',6-dihydroxyspirojisobenzofuran-1 (3H),9'[9H]xanthen]-3-on-dinatriumsalz (C145410; Acid Red No. 92), 3',6'-Dihydroxy-4',5'-diiodospiro-[isobenzofuran-1 (3H),9'(9H)-xanthen]-3-on-dinatriumsalz (Cl45425; Acid Red No. 95), (2-Sulfophenyl)di[4-(ethyl((4-sulfophenyl)methyl)amino)-phenyl]-carbenium-dinatriumsalz, betain (Cl42090; Acid Blue No. 9; FD&C Blue No. 1), 1,4-Bis[(2-sulfo-4-methylphenyl)amino]-9,10-anthrachinon-dinatriumsalz (Cl 61570; Acid Green No. 25), Bis[4-(dimethylamino)phenyl]-(3,7-disulfo-2-hydroxynaphth-1-yl) carbenium-inneres Salz, mononatriumsalz (Cl44090; Food Green No. 4; Acid Green No. 50), Bis[4-(diethylamino)phenyl](2,4-disulfophenyl)-carbenium-inneres salz, Natriumsalz (2:1) (Cl42045; Food Blue No. 3; Acid Blue No. 1), Bis[4-(diethylamino)phenyl](5-hydroxy-2,4-disulfophenyl)carbenium-inneres salz, Calciumsalz (2:1) (Cl42051; Acid Blue No. 3), 1-Amino-4-(cyclohexylamino)-9,10-anthrachinon-2-sulfonsäure-natriumsalz (Cl62045; Acid Blue No. 62), 2-(1,3-Dihydro-3-oxo-5-sulfo-2H-indol-2-yliden)-2,3-dihydro-3-oxo-1 H-indol-5-sulfonsäure-dinatriumsalz (CI73015; Acid Blue No. 74), 9-(2-Carboxyphenyl)-3-[(2-methylphenyl)-amino]-6-[(2-methyl-4-sulfophenyl)amino]xanthylium-inneres Salz, mononatriumsalz (Cl45190; Acid Violet No. 9), 1-Hydroxy-4-[(4-methyl-2-sulfophenyl)amino]-9,10-anthrachinon-natriumsalz (Cl60730; D&C Violett No. 2; Acid Violet No. 43), Bis[3-nitro-4-[(4-phenylamino)-3-sulfo-phenyl-amino]-phenyl]-sulfon (Cl10410; Acid Brown No. 13), 5-Amino-4-hydroxy-6-[(4-nitrophenyl)azo]-3-(phenylazo)-2,7-naphthalin-disulfonsäure-dinatriumsalz (Cl20470; Acid Black No. 1), 3-Hydroxy-4-[(2-hydroxy-naphth-1-yl)azo]-7-nitro-1-naphthalin-sulfonsäure-chromkomplex (3:2) (Cl15711; Acid Black No. 52), 3-[(2,4-Dimethyl-5-sulfophenyl)azol-4-hydroxy-1-naphthalin-sulfonsäure-dinatriumsalz (Cl14700; Food Red No. 1; Ponceau SX; FD&C Red No. 4), 4-(Acetylamino)- 5-hydroxy- 6-[(7-sulfo- 4-[(4-sulfophenyl) azo] naphth- 1-yl) azo]- 1,7-naphthalindisulfonsäure-tetranatriumsalz (Cl28440; Food Black No. 1) und 3-Hydroxy-4-(3-methyl-5-oxo-1-phenyl-4,5-dihydro-1 H-pyrazol-4-ylazo)-naphthalin-1-sulfonsäure-natriumsalz, Chrom-Komplex (Acid Red No. 195), insbesondere 2,6-Diamino-3-(pyridin-3-yl)azo-pyridin, 8-Amino-2-brom-5-hydroxy-4-imino-6-[(3-(trimethylammonio)phenyl)amino]-1(4H)-naphthalinon-chlorid (Cl56059; Basic Blue No. 99), oder Nitrofarbstoffe, beispielsweise 1,4-Bis[(2-hydroxyethyl)amino]-2-nitrobenzol, 1-(2-Hydroxyethyl) amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10). 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] - 2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol,4-[(3-Hydroxypropyl) amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxy-ethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)-amino]-1-methoxy-5-nitrobenzol, 2-Amino-3-nitrophenol, 1-(2-Hydroxy-ethoxy)-3-methylamino-4-nitrobenzol, 2,3-(Dihydroxypropoxy)-3-methyl-amino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl) amino]-3-nitro-benzamid (HC Yellow No. 15), enthalten. Besonders bevorzugte direktziehende Farbstoffe sind hierbei 4-Amino-1-[(2-hydroxyethyl)amino]-2-nitrobenzol (HC Red No. 3), 1-Amino-4-[di(2-hydroxyethyl)amino]-2-nitrobenzol-hydrochlorid (HC Red No. 13), 8-Amino-2-brom-5-hydroxy-4-imino-6-[(3-(trimethylammonio)-phenyl)-amino]-1 (4H)-naphthalinon-chlorid (Cl56059; Basic Blue No. 99), 4-[Ethyl-(2-hydroxyethyl)amino]-1-[(2-hydroxyethyl)amino]-2-nitrobenzol-hydrochlorid (HC Blue No. 12), 1-(2-Hydroxyethyl)-amino-2-nitro-4-[di(2-hydroxyethyl)amino]-benzol, 4-[Ethyl-(2-hydroxyethyl)amino]-1-[(2-hydroxyethyl)amino]-2-nitrobenzol-hydrochlorid, 4-Amino-3-nitro-phenol, 1-Amino-4-[di(2-hydroxyethyl)amino]-2-nitrobenzol-hydrochlorid und/oder 2-Amino-6-chlor-4-nitro-phenol sowie 2,6-Diamino-3-(pyridin-3-yl)azo-pyridin.

Die direktziehenden Farbstoffe können in dieser Farbträgermasse in einer Menge von etwa 0,01 bis 10 Gewichtsprozent, vorzugsweise 0,1 bis 5 Gewichtsprozent, eingesetzt werden.

In dem Mehrkomponenten-Kit befindet sich getrennt von der Farbträgermasse auch das Oxidationsmittel. Die Menge des in dem Mehrkomponenten-Kit enthaltenen Wasserstoffperoxids oder der Wasserstoffperoxid-Additionsprodukte oder der oxidierend wirkenden Enzyme ist so bemessen, daß sie ausreicht, um die Mischung der Farbstoffvorstufen quantitativ in den Oxidationsfarbstoff umzusetzen. Dabei kann das Oxidationsmittel entweder in gebrauchsfertiger Form oder als Trockensubstanz vorliegen, die nach Zusatz eines geeigneten Lösungsmittels angewendet werden kann.

Als Oxidationsmittel, das ebenfalls in dem erfindungsgemäßen Mehrkomponenten-Kit enthalten ist, werden in der Regel Wasserstoffperoxid oder dessen Additionsverbindungen an Harnstoff, Melamin oder Natriumbromat verwendet, wobei Wasserstoffperoxid besonders bevorzugt ist. Im allgemeinen werden Wasserstoffperoxid oder die Wasserstoffperoxid-Additionsprodukte zur Oxidation der Farbstoffvorstufen in einer Konzentration von 1 bis 12 Gewichtsprozent eingesetzt.

Haarschonender ist jedoch die enzymatische Oxidation der Farbstoffvorstufen mit Hilfe von Luft oder Sauerstoff. Sie zeichnet sich durch besonders milde Bedingungen aus. Der pH-Wert liegt im schwach sauren bis schwach alkalischen Bereich und die verwendeten Enzymproteine greifen die Haarstruktur nicht an. Im Gegensatz zur Anwendung von Peroxiden ist jedoch bei der Anwendung von oxidierend wirkenden Enzymen ein "Hellerfärben" der Haare nicht möglich.

Für die oxidative Erzeugung von Oxidationsfarben mit Hilfe von Luft oder Sauerstoff in Gegenwart von Enzymen stehen einstufige oder mehrstufige enzymatische Oxidationssysteme zur Verfügung. Bei den einstufigen Enzymsystemen können aromatische Phenole und Amine in einer Färbemischung unter Sauerstoffzufuhr ohne Peroxidzusatz direkt zum polymeren Farbstoff oxidiert werden. Hierfür sind Phenoloxidasen, vorzugsweise Laccasen, geeignet. Im Gegensatz hierzu werden bei den mehrstufigen enzymatischen Oxidationssystemen mehrere Enzyme für die Farbstoffproduktion benötigt.

Für ein mehrstufiges, enzymatisches Oxidationssystem zur Herstellung des Oxidationsfarbstoffes aus den Farbvorstufen kann eine Kombination eines Sauerstoff-Oxidoreductase/Substrat-Systems und einer Peroxidase angewendet werden. Beispiele für ein Sauerstoff-Oxidoreductase/Substrat-System sind folgende:

Glucose-Oxidase (EC 1.1.3.4)/D-Glucose
Alkohol-Oxidase (EC 1.1.3.13)/Ethanol
Pyruvat-Oxidase (EC 1.2.3.3)/Pyruvat
Oxalat-Oxidase (EC 1.2.3.4)/Oxalat
Cholesterin-Oxidase (EC 1.1.3.6)/Cholesterin
Uricase (EC 1.7.3.3)/Harnsäure
Lactat-Oxidase/Milchsäure
Xanthin-Oxidase (EC 1.1.3.22)/Xanthin.

Die für die Enzyme in Klammern angegebenen Klassifizierungen erfolgen gemäß der "Classification of the International Union of Biochemistry on Nomenclature and Classification of Enzymes (1984)".

Die Zubereitungsform für die Farbträgermasse sowie für das gebrauchsfertige Oxidationsfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Die in dem erfindungsgemäßen Mehrkomponenten-Kit enthaltenen Mittel zur Erzeugung einer nicht-oxidativen Färbung (Komponente (I)) enthalten als Farbstoffe die vorgenannten direktziehenden Farbstoffe, wobei diese Farbstoffe in einer Gesamtmenge von etwa 0,01 bis 10 Gewichtsprozent, vorzugsweise 0,1 bis 5 Gewichtsprozent, eingesetzt werden.

Das nicht-oxidative Färbemittel kann beispielsweise in Form einer Lösung, insbesondere einer wäßrigen oder wäßrig-alkoholischen Lösung vorliegen. Die besonders bevorzugten Zubereitungsformen sind jedoch eine Creme, ein Gel, ein Aerosolschaum oder eine Emulsion. Ihre Zusammensetzung stellt eine Mischung der Farbstoffkomponenten mit den für solche Zubereitungen üblichen Zusätzen dar.

Übliche in oxidativen beziehungsweise nicht-oxidativen Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Der pH-Wert des gebrauchsfertigen oxidativen beziehungsweise nicht-oxidativen Färbemittels beträgt in der Regel 3 bis 11, vorzugsweise 5 bis 9.

Der pH-Wert des gebrauchsfertigen Oxidationsfärbemittels stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis beinflußt wird.

Zur Einstellung des für die Färbung geeigneten pH-Wertes können alkalisierende Mittel wie Alkanolamine, Alkylamine, Alkalihydroxide oder Ammoniumhydroxid und Alkalicarbonate oder Ammoniumcarbonate, vorzugsweise Ammoniumhydroxid, oder Säuren wie Milchsäure, Essigsäure, Weinsäure, Phosphorsäure, Salzsäure, Zitronensäure, Ascorbinsäure und Borsäure, verwendet werden.

Insbesondere bei der enzymatisch katalysierten Oxidation empfiehlt sich zur Kontrolle des pH-Wertes die Verwendung eines Puffersystems. Dabei können Zitratpuffer, Phosphatpuffer oder Boratpuffer eingesetzt werden. Bevorzugt ist die Verwendung eines Boratpuffers (Borsäure/NaOH) oder eines Phosphatpuffers ($KH_2PO_4/K_2HPO_4$).

Im Falle der oxidativen Färbung wird unmittelbar vor der Anwendung eines der vorstehend genannten Oxidationsmittel mit der die Farbstoffvorstufen und gegebenenfalls direktziehende Farbstoffe sowie die übrigen Hilfsmittel enthaltenden Farbträgermasse vermischt und auf das Haar aufgetragen. Je nach gewünschter Farbtiefe läßt man diese Mischung 5 bis 60 Minuten, vorzugsweise 15 bis 30 Minuten, bei einer Temperatur von 20 bis 50 Grad Celsius, insbesondere bei 30 bis 40 Grad Celsius einwirken. Anschließend wird das Haar mit Wasser gespült und gegebenenfalls mit einem Shampoo gewaschen.

Die Farbträgermasse und das Oxidationsmittel werden hierbei im Gewichtsverhältnis von 5:1 bis 1:3 miteinander vermischt, wobei ein Gewichtsverhältnis von 1:1 bis 1:2 besonders bevorzugt ist.

Im Falle der nicht-oxidativen Färbung wird das Färbemittel auf das Haar aufgetragen. Je nach gewünschter Farbtiefe läßt man diese Mischung sodann 5 bis 60 Minuten, vorzugsweise 15 bis 30 Minuten, bei einer Temperatur von 20 bis 50 Grad Celsius, insbesondere bei 30 bis 40 Grad Celsius einwirken. Anschließend wird das Haar mit Wasser gespült und gegebenenfalls mit einem Shampoo gewaschen.

Ein weiterer wesentlicher Bestandteil des erfindungsgemäßen Mehrkomponenten-Kits ist das Mittel zur reduzierenden Entfärbung der mit Oxidationsfarbstoffen und/oder direktziehenden Farbstoffen gefärbten Fasern der Komponente (II), welches eine Kombination aus a) mindestens einem Redukton und/oder einem Thiol und/oder einem Sulfit und b) mindestens einer $\alpha$-Oxocarbonsäure oder derem physiologisch verträglichem Salz enthält.

Als Reduktone können zum Beispiel Ascorbinsäure oder Isoascorbinsäure beziehungsweise deren Salze oder Ester, beispielsweise 6-O-Palmitoylascorbat, Hydroxypropandial (Triosereduktion), 2,3-Dihydroxy-2-cyclopenten-1-on (Reduktinsäure) oder Mischungen dieser Verbindungen, vorzugsweise in einer Menge von 1 bis 50 Gewichtsprozent, insbesondere von 2 bis 10 Gewichtsprozent, eingesetzt werden, wobei die Verwendung von Ascorbinsäure oder Isoascorbinsäure und insbesondere von Ascorbinsäure bevorzugt ist. Bei Verwendung von Ascorbinsäuresalzen oder Isoascorbinsäuresalzen kann die freie Säure auch in situ aus den Salzen, beispielsweise den Alkalimetallascorbaten oder Erdalkalimetallascorbaten beziehungsweise den Alkalimetallisoascorbaten oder Erdalkalimetallisoascorbaten, durch Zusatz einer Säure erzeugt werden. Dies ist wegen der besseren Löslichkeit der Salze in Wasser insbesondere bei höheren Konzentrationen von Vorteil. Als Ascorbinsäuresalze oder Isoascorbinsäuresalze kommen hierbei insbesondere das Calciumsalz, das Magnesiumsalz und das Natriumsalz der Ascorbinsäure oder Isoascorbinsäure in Betracht.

Als Thiole können Cystein oder dessen Salze, N-Acetylcystein, Cysteamin oder dessen Salze, Mercaptoacetaldehyd, Penicillamin, Glutathion, Homocystein oder dessen Salze und/oder Calciumthioglykolat. eingesetzt werden, wobei Cystein und dessen Salze besonders bevorzugt werden.

Weiterhin kann das Entfärbemittel Sulfite, beispielsweise Alkalisulfite oder Erdalkalisulfite, insbesondere Natriumsulfit, enthalten um eine Rückoxidation der eventuell im Haar verbleibenden Farbstoffvorstufen zu verhindern.

Die Einsatzmenge an Thiol beträgt 0,1 bis 10 Gewichtsprozent, vorzugsweise 2 bis 5 Gewichtsprozent, während das Sulfit in einer Menge von 0,001 bis 5 Gewichtsprozent, vorzugsweise in einer Menge von 0,01 bis 0,5 Gewichtsprozent eingesetzt wird.

Als $\alpha$-Oxocarbonsäure können sowohl $\alpha$-Aldehydsäuren als auch $\alpha$-Ketocarbonsäuren, wie zum Beispiel Glyoxylsäure, Brenztraubensäure, Oxalessigsäure oder $\alpha$-Ketoglutarsäure, sowie deren Alkalisalze und Erdalkalisalze verwendet werden.

Die Einsatzmenge an $\alpha$-Oxocarbonsäure beziehungsweise derem physiologisch verträglichen Salz beträgt 0,1 bis 10 Gewichtsprozent, wobei eine Einsatzmenge von 0,5 bis 5 Gewichtsprozent bevorzugt ist.

Die entsprechenden $\alpha$-Oxocarbonsäureester verbessern die Entfärbereaktion nicht und können daher nur nach einer (beispielsweise in-situ) Esterverseifung als $\alpha$-Oxocarbonsäuren eingesetzt werden.

In einer besonders bevorzugten Ausführungsform enthält das Entfärbemittel als Komponente a) eine Kombination aus mindestens einem Redukton, vorzugsweise Ascorbinsäure und mindestens einem Thiol, vorzugsweise Cystein und/oder Cystein-Hydrochlorid, und als Komponente b) eine $\alpha$-Oxocarbonsäure oder deren physiologisch verträgliches Salz.

Es ist jedoch ebenfalls möglich, zur Entfärbung ein Mittel zu verwenden, welches ein Redukton oder ein Thiol oder ein Sulfit alleine, oder eine Kombination aus einem Thiol und einem Sulfit beziehungsweise eine Kombination aus einem Redukton und einem Sulfit oder einem Redukton und einem Thiol und einem Sulfit und als Komponente b) eine $\alpha$-Oxocarbonsäure oder deren physiologisch verträgliches Salz enthält.

Das Mittel zur reduzierenden Entfärbung der mit einer Kombination von Oxidationsfarbstoffen und/oder direktziehenden Farbstoffen gefärbten Fasern (im folgenden "Entfärbemittel" genannt) kann als wäßrige oder wäßrig-alkoholische Lösung, als Gel, Creme, Emulsion oder Schaum vorliegen, wobei das Entfärbemittel sowohl in Form eines Einkomponentenpräparats als auch in Form eines Mehrkomponentenpräparates konfektioniert sein kann. Das Entfärbemittel kann neben der Pulverform zum Schutz vor Staubbildung auch als Tablette - auch Brausetablette - oder Granulat konfektioniert sein. Hieraus wird dann vor der Anwendung mit kaltem oder warmem Wasser, gegebenenfalls unter Zusatz eines oder mehrerer der nachfolgend genannten Hilfsmittel, das Entfärbemittel hergestellt. Es ist jedoch auch möglich, daß diese Hilfsmittel (sofern sie in fester Form vorliegen) bereits in dem Entfärbepulver oder Entfärbegranulat beziehungsweise der Brausetablette enthalten sind. Durch Benetzung des Pulvers durch Öle oder Wachse kann zusätzlich die Staubbildung vermindert werden.

Das Entfärbemittel kann zusätzliche Hilfsmittel, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain, enthalten.

Der pH-Wert des Entfärbemittels beträgt etwa 1,8 bis 6, insbesondere 2,5 bis 4. Erforderlichenfalls kann der gewünschte pH-Wert durch Zugabe von weiteren Säuren, beispielsweise α-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Alkanolaminen, Alkylaminen, Alkalihydroxiden, Ammoniumhydroxiden, Alkalicarbonaten, Ammoniumcarbonaten oder Alkaliphosphaten, eingestellt werden.

Obwohl es besonders einfach ist, die α-Oxocarbonsäure direkt dem Entfärbemittel zuzusetzen, ist es ebenfalls ohne Verschlechterung des Entfärbeergebnisses möglich, die α-Oxocarbonsäure von dem Entfärbemittel getrennt abzupacken und im Anschluß an die Entfärbebehandlung in einem gesonderten Nachbehandlungsschritt einzusetzen.

Ebenfalls ist es möglich, gleichzeitig sowohl im Entfärbemittel als auch im Nachbehandlungsmittel eine α-Oxocarbonsäure oder deren physiologisch verträgliches Salz einzusetzen.

Überraschenderweise wurde festgestellt, daß das Entfärbeergebnis unmittelbar nach der Entfärbebehandlung zwar sowohl ohne α-Oxocarbonsäurezusatz (das heißt bei alleiniger Verwendung der Komponente a) ) als auch mit α-Oxocarbonsäurezusatz annähernd gleich ist; nach einiger Zeit jedoch deutliche Unterschiede auftreten. So verfärben sich die ohne α-Oxocarbonsäurezusatz entfärbten Haare nach einigen Stunden oder Tagen infolge der Luftoxidation wieder teilweise, während die mit α-Oxocarbonsäurezusatz entfärbten Haare unverändert bleiben. Der α-Oxocarbonsäurezusatz bewirkt somit eine Fixierung des Entfärbeergebnisses und verhindert die Rückbildung der entfernten Färbung.

Die Einwirkungszeit des Entfärbemittels beträgt je nach zu entfärbender Färbung und Temperatur (etwa 20 bis 50 Grad Celsius) 5 bis 60 Minuten, insbesondere 15 bis 30 Minuten, wobei durch Wärmezufuhr der Entfärbeprozeß beschleunigt werden kann. Nach Beendigung der Einwirkungszeit des Entfärbemittels wird das Haar mit Wasser gespült, gegebenenfalls mit einem Shampoo gewaschen.

Enthält das Entfärbemittel keine α-Oxocarbonsäure wird das Haar im Anschluß an die Entfärbebehandlung mit einer α-Oxocarbonsäurenhaltigen Spülung, vorzugsweise im sauren pH-Bereich, nachbehandelt und sodann getrocknet.

Die Anwendung des erfindungsgemäßen Entfärbemittels der Komponente (II) ist natürlich nicht auf die Entfärbung der mit der Komponente (I) des erfindungsgemäßen Mehrkomponenten-Kits erzeugten Färbungen beschränkt. Vielmehr kann die Entfärbezubereitung der Komponente (II) ganz allgemein zur Entfärbung von Färbungen eingesetzt werden, auch wenn diese nicht mit Hilfe des erfindungsgemäßen Färbemittels der Komponente (I) , sondern auf einem ganz anderen und unabhängigen Weg erzeugt wurden. Darüber hinaus eignet sich die erfindungsgemäße Entfärbezubereitung der Komponente (II) auch zur Entfärbung einer Vielzahl von anderen natürlichen oder synthetischen Fasern, wie zum Beispiel Baumwolle, Seide, Viskose, Nylon, Celluloseacetat, sofern diese mit Oxidationsfarbstoffen und/oder direktziehenden Farbstoffen gefärbt worden sind, und ist nicht auf die Entfärbung von Keratinfasem, beispielsweise Wolle, Pelzen oder menschlichen Haaren, beschränkt.

Gegenstand der vorliegenden Anmeldung ist daher auch ein Mittel zur reduzierenden Entfärbung von mit Oxidationsfarbstoffen und/oder direktziehenden Farbstoffen gefärbten Fasern, insbesondere Haaren, welches eine Kombination aus a) mindestens einem Redukton, wie zum Beispiel Ascorbinsäure oder Isoascorbinsäure beziehungsweise deren Salzen oder Estem, beispielsweise 6-O-Palmitoyl-ascorbinsäure, Hydroxypropandial (Trioseredukton), 2,3-Dihydroxy-2-cyclopenten-1-on (Reduktinsäure) oder Mischungen dieser Verbindungen, insbesondere Ascorbinsäure, und/oder mindestens einem Thiol, insbesondere Cystein oder dessen Salzen, und/oder mindestens einem Sulfit, insbesondere Natriumsulfit, und b) mindestens einer α-Oxocarbonsäure oder derem physiologisch verträglichem Salz enthält, sowie die Verwendung dieses Mittels zur Entfärbung von mit Oxidationsfarbstoffen und/oder direktziehenden Farbstoffen gefärbten Fasern, insbesondere Haaren. Besonders bevorzugt ist hierbei die Verwendung einer Kombination von a) mindestens einem Redukton, insbesondere Ascorbinsäure oder Isoascorbinsäure oder deren Salzen, und mindestens einem Thiol, insbesondere Cystein und/oder Cystein-Hydrochlorid, und b) mindestens einer α-Oxocarbonsäure oder derem physiologisch verträglichem Salz.

Das erfindungsgemäße Entfärbemittel ermöglicht eine schnelle, schonende, gleichmäßige und haltbare Entfärbung von mit Oxidationsfarbstoffen und/oder direktziehenden Farbstoffen gefärbten Fasern ohne Restverfärbungen der Faser.

Die nachfolgenden Beispiele sollen den Gegenstand näher erläutern, ohne ihn auf diese Beispiele zu beschränken.

**Beispiele**

**Beispiele 1.1 bis 1.14:**

**a. Oxidationshaarfärbemittel**

| | |
|---|---|
| Entwicklersubstanz(en) (gegebenenfalls mit NH$_3$ (25%ige wäßrige Lösung) oder NaOH (10%ige wäßrige Lösung) versetzen) | Mengenangaben gemäß Tabelle 1 |
| Kupplersubstanz(en) (gegebenenfalls mit NH$_3$ (25 %ige wäßrige Lösung) oder NaOH (10 %ige wäßrige Lösung) versetzen) | Mengenangaben gemäß Tabelle 1 |
| Nitrofarbstoffe | Mengenangaben gemäß Tabelle 1 . |
| Dinatrium-ethylendiaminotetraacetat | 0,30 g |
| Natriumsulfit | 0,40 g |
| Natriumlaurylethersulfat (28 %ige wäßrige Lösung) | 10,00 g |
| Isopropanol | 10,00 g |
| Ammoniak (25 %ige wäßrige Lösung) | 9,10 g |
| Wasser, vollentsalzt | ad 100,00 g |

5g der vorstehenden Farbträgermasse werden mit 5g einer 4 %igen Wasserstoffperoxidlösung vermischt. Das erhaltene gebrauchsfertige Oxidationshaarfärbemittel wird auf die Haare aufgetragen und mit einem Pinsel gleichmäßig verteilt. Nach einer Einwirkungszeit von 30 Minuten bei 40 °C wird das Haar mit lauwarmem Wasser gespült und sodann getrocknet.

**b1. Entfärbegel:**

| | |
|---|---|
| Ascorbinsäure | 5,00 g |
| Methylhydroxyethylcellulose (Tylose MHB 10.OOOP der Firma HoechstlBRD) | 1,50 g |
| Cystein | 2,00 g |
| Glyoxylsäure | 0,50 g |
| Trinatrium Citrat Dihydrat | 0,30 g |
| Wasser | ad 100,00 g |

**b2. Entfärbegel:**

| | |
|---|---|
| Isoascorbinsäure | 5,00 g |

Tabelle fortgesetzt

| Methylhydroxyethylcellulose (Tylose MHB 10.000P der Firma Hoechst/BRD) | 1,50 g |
|---|---|
| Cystein | 2,00 g |
| Natriumsulfit | 0,05 g |
| Glyoxylsäure | 0,50 g |
| Trinatrium Citrat Dihydrat | 0,20 g |
| Wasser | ad 100,00 g |

**b3. Entfärbegel:**

| Ascorbinsäure | 10,00 g |
|---|---|
| Hydroxyethylcellulose | 1,50 g |
| Glutathion | 1,00 g |
| Glyoxylsäure | 0,50 g |
| Trinatrium Citrat Dihydrat | 0,90 g |
| Wasser | ad 100,00 g |

**b4. Entfärbegel:**

| Natriumascorbat | 6,00 g |
|---|---|
| Citronensäure | 6,00 g |
| Hydroxyethylcellulose | 1,50 g |
| Glyoxylsäure | 0,50 g |
| Wasser | ad 100,00 g |

Der pH-Wert des Entfärbegels liegt zwischen 2,5 und 3,5.

**b5. Entfärbegel:**

| Isoascorbinsäure | 6,00 g |
|---|---|
| Methylhydroxyethylcellulose (Tylose MHB 10.OOOP der Firma Hoechst/BRD) | 1,50 g |
| Cystein | 2,00 g |
| $\alpha$-Ketoglutarsäure | 0,80 g |
| Wasser | ad 100,00 g |

Der pH-Wert wird mit Trinatrium Citrat Dihydrat auf 2.5 bis 3.0 eingestellt.

**b6. Entfärbegel:**

| Natriumascorbat | 5,70 g |
|---|---|
| L-Cystein | 2,00 g |
| Magnesiumsulfat | 1,00 g |
| Citronensäure | 7,40 g |
| Trinatriumcitrat Dihydrat | 1,00 g |
| Hydroxyethylcellulose | 1,50 g |

Tabelle fortgesetzt

| 2-Oxoglutarsäure | 0,80 g |
|---|---|
| Wasser | ad 100,00 g |

**b7. Entfärbegel:**

| Natriumascorbat | 5,70 g |
|---|---|
| L-Cystein | 2,00 g |
| Magnesiumsulfat | 1,00 g |
| Citronensäure | 7,40 g |
| Trinatriumcitrat Dihydrat | 0,60 g |
| Hydroxyethylcellulose | 1,50 g |
| Glyoxylsäure | 0,50 g |
| Wasser | ad 100,00 g |

**b8. Entfärbegel:**

| Natriumascorbat | 5,70 g |
|---|---|
| L-Cystein | 2,00 g |
| Magnesiumsulfat | 1,00 g |
| Citronensäure | 7,40 g |
| Trinatriumcitrat Dihydrat | 1,00 g |
| Hydroxyethylcellulose | 1,50 g |
| Oxalessigsäure | 0,70 g |
| Wasser | ad 100,00 g |

Auf das gefärbte Haar trägt man das oben beschriebene Entfärbegel auf und läßt es jeweils 30 Minuten bei 37 Grad Celsius (Entfärbemittet b1 bis b3) beziehungsweise 60 Minuten bei 40 Grad Celsius (Entfärbemittel b4 und b5) beziehungsweise 20 bis 60 Minuten bei 25 bis 30 Grad Celsius (Entfärbemittel b6) unter einer Plastikabdeckung einwirken, danach wird gründlich mit Wasser und einem Shampoo gewaschen, gegebenenfalls mit Wasser gespült und sodann getrocknet.

Das Ergebnis dieser Entfärbehandlung ist in Tabelle 1 zusammengefaßt.

## Tabelle 1: Färbe- und Entfärbe-Resultate

| Nr. | Entwickler/Kuppler-Kombination | Farbton nach dem Färben | Farbmeßwerte | | | Entfärbe-% |
|-----|-------------------------------|------------------------|------|------|------|-----------|
|     |                               |                        | L    | a    | b    |           |
| 1.1 | 1,4-Diamino-2-(2-hydroxyethyl)-benzol sulfat: 0,62 g <br> 1,4-Diamino-2-methyl-benzol sulfat: 0,55 g <br> 5-Amino-2-methyl-phenol: 0,61 g | tiefviolett | unbehandelte Haare: <br> 37,29; +8,13; +15,88 <br><br> Nach dem Färben: <br> 25,24; +12,32; +3,35 <br><br> nach 1x Entfärben mit **b2:** <br> 35,95; +9,42; +14,42 | | | 87 |

EP 0 943 316 B1

Tabelle 1 (Fortsetzung)

| Nr. | Entwickler/Kuppler-Kombination | Farbton nach dem Färben | Farbmeßwerte | | | Entfärbe-% |
|-----|-------------------------------|------------------------|--------------|---|---|------------|
| | | | L | a | b | |
| 1.2 | 4-Amino-2-(aminomethyl)-phenol hydrochlorid: 1,05 g 5-Amino-2-methyl-phenol: 0,61 g | orange | unbehandelte Haare: 37,29; +8,13; +15,88 Nach dem Färben: 30,22; +14,32; +14,00 nach 1x Entfärben mit b1: 37,57; +9,35; +16,71 | | | 84 |

EP 0 943 316 B1

## Tabelle 1 (Fortsetzung)

| Nr. | Entwickler/Kuppler-Kombination + Nitrofarbstoffe | Farbton nach dem Färben | Farbmeßwerte | | | Entfärbe-% |
|-----|--------------------------------------------------|-------------------------|---|---|---|------------|
| | | | L | a | b | |
| 1.3 | 4-Amino-3-methyl phenol: 0,61 g | violett | unbehandelte Haare: | | | |
| | 1-Naphthol: 0,36 g | | 37,29; | +8,13; | +15,88 | |
| | 5-Amino-2-methyl-phenol: 0,31 g | | | | | |
| | 4-[Ethyl-(2-hydroxyethyl)amino]1- | | Nach dem Färben: | | | |
| | [2(2-hydroxyethyl)amino]-2-nitro- | | 30,42; | +10,41; | +7,99 | |
| | benzol-hydrochlorid (HC Blue No. | | | | | |
| | 12): 0,5 g | | nach 1x Entfärben mit b1: | | | 81 |
| | | | 37,72; | +9,30; | +14,26 | |

EP 0 943 316 B1

## Tabelle 1 (Fortsetzung)

| Nr. | Entwickler/Kuppler-Kombination Nitrofarbstoff(e) | Farbton nach dem Färben | Farbmeßwerte | | | Entfärbe-% |
|-----|--------------------------------------------------|-------------------------|--------------|---|---|------------|
| | | | L | a | b | |
| 1.4 | 4-Amino-3-methylphenol: 1,92 g | blauviolett | unbehandelte Haare: | | | |
| | 1-Naphthol: 0,32 g | | 34,41; +7,27; +13,78 | | | |
| | 2-Amino-4[(2-hydroxyethyl)amino]- | | | | | |
| | anisol sulfat: 0,61 g | | Nach dem Färben: | | | |
| | 5-Amino-2-methylphenol: 1,38 g | | 22,82; +8,86; +3,87 | | | |
| | | | | | | 86 |
| | HC Blue 12: 1,00 g | | nach 1x Entfärben mit b3: | | | |
| | | | 35,38; +8,95; +12,92 | | | |

EP 0 943 316 B1

## Tabelle 1 (Fortsetzung)

| Nr. | Entwickler/Kuppler-Kombination Nitrofarbstoff(e) | Farbton nach dem Färben | Farbmeßwerte | | | Entfärbe-% |
|---|---|---|---|---|---|---|
| | | | L | a | b | |
| 1.5 | 1,4-Diamino-2-methyl-benzolsulfat: 2,2g 5-Amino-2-methylphenol: 1,23g | tiefviolett | unbehandelte Haare: 34,41; +7,27; +13,78 Nach dem Färben: 20.04; +7,55; +0,08 nach 1x Entfärben(60 min, 40 °C) mit b4: 31,85; +9,28; +14,54 | | | 83 |

EP 0 943 316 B1

## Tabelle 1 (Fortsetzung)

| Nr. | Entwickler/Kuppler-Kombination Nitrofarbstoff(e) | Farbton nach dem Färben | Farbmeßwerte | | | Entfärbe-% |
|---|---|---|---|---|---|---|
| | | | L | a | b | |
| 1.6 | 4,5-Diamino-1-(2-hydroxyethyl)-1H-pyrazol: 1,2 g  5-Amino-2-methylphenol: 0,62 g | intensiv orange-rot | unbehandelte Haare: 34,41; +7,27; +13,78  Nach dem Färben: 27,66; +23,98; +15,06  nach 1x Entfärben(60 min, 40 °C) mit b4: 33,83; +10,92; +15,08 | | | 78 |

EP 0 943 316 B1

EP 0 943 316 B1

## Tabelle 1 (Fortsetzung)

| Nr. | Entwickler/Kuppler-Kombination Nitrofarbstoff(e) | Farbton nach dem Färben | Farbmeßwerte | | | Entfärbe-% |
|-----|---------------------------------------------------|-------------------------|------|------|------|------------|
|     |                                                   |                         | L    | a    | b    |            |
| 1.7 | 1,4-Diamino2-(2-hydroxyethyl)-benzol-sulfat: 1,25 g 4-(2-Hydroxyethoxy)-1,3-phenylen-diamin-dihydrochlorid: 1,20 g | blau-schwarz | unbehandelte Haare: 34,41; +7,27; +13,78 Nach dem Färben: 19,76; +0,70; -2,15 nach 1x Entfärben (60 min, 40 °C) mit b4: 32,21; +10,31; +12, 98 | | | 83 |

## Tabelle 1 (Fortsetzung)

| Nr. | Entwickler/Kuppler-Kombination Nitrofarbstoff(e) | Farbton nach dem Färben | Farbmeßwerte | | | Entfärbe-% |
|---|---|---|---|---|---|---|
| | | | L | a | b | |
| **1.8** | 4-Amino-3-methylphenol: 1,92 g | rotbraun | unbehandelte Haare: | | | |
| | 1-Naphthol: 0,32 g | | 34,41; | +7,27; | +13,78 | |
| | 5-Amino-2-methylphenol: 1,38 g | | Nach dem Färben: | | | |
| | 2-Amino-4[(2-hydroxyethyl)amino]- | | 28,18; | +15,19; | +11,12 | |
| | anisol sulfat: 0,61 g | | nach 1x Entfärben(60 min, 40 °C): | | | 75 |
| | | | mit **b4**: | | | |
| | | | 34,65; | +9,63; | +14,76 | |

EP 0 943 316 B1

EP 0 943 316 B1

## Tabelle 1 (Fortsetzung)

| Nr. | Entwickler/Kuppler-Kombination Nitrofarbstoff(e) | Farbton nach dem Färben | Farbmeßwerte | | | Entfärbe-% |
|---|---|---|---|---|---|---|
| | | | L | a | b | |
| 1.9 | 1,4-Diamino-2-methylbenzol-sulfat: 0,55 g 2-Methyl-1-naphthol-acetat: 0,5 g 5-Amino-2-methylphenol: 0,31 g | tiefviolett | unbehandelte Haare: 34,41; +7,27; +13,78 Nach dem Färben: 20,74; +7,91; -0,53 nach 1x Entfärben (60 min, 40 °C): 90 mit b4: 35,20; +9,05; +14,40 | | | |

## Tabelle 1 (Fortsetzung)

| Nr. | Entwickler/Kuppler-Kombination Nitrofarbstoff(e) | Farbton nach dem Färben | Farbmeßwerte | | | Entfärbe-% |
|---|---|---|---|---|---|---|
| | | | L | a | b | |
| 1.10 | 1,4-Diamino-2-(2-hydroxyethyl)-benzol-sulfat: 1,2 g <br> 5-((2,2,2-Trifluorethyl)-amino)-2-methyl-phenol: 1,0 g | violett | unbehandelte Haare: <br> 84,20;   -1,36;   +8,81 <br> Nach dem Färben: <br> 25,22;   +18,36;   -4,83 <br> nach 1x Entfärben (60 min, 40 °C):   69 <br> mit b4: <br> 70,83;   +9,59;   +19,81 | | | |

**Tabelle 1 (Fortsetzung)**

| Nr. | Entwickler/Kuppler-Kombination Nitrofarbstoff(e) | Farbton nach dem Färben | Farbmeßwerte | | | Entfärbe-% |
|-----|---|---|---|---|---|---|
| | | | L | a | b | |
| 1.11 | 1,4-Diamino-2-methylbenzol-sulfat: 0,55g<br>4,5-Diamino-1-(2-hydroxyethyl)-1H-pyrazol: 0,6 g<br>5-Amino-2-methylphenol: 0,62 g | intensiv rot | unbehandelte Haare:<br>34,41; +7,27; +13,78<br>Nach dem Färben:<br>21,13; +14,99; +5,29<br>nach 1x Entfärben (60 min, 40 °C): 74<br>mit **b4**:<br>35,65; +10,35; +16,84 | | | |

EP 0 943 316 B1

EP 0 943 316 B1

## Tabelle 1 (Fortsetzung)

| Nr. | Entwickler/Kuppler-Kombination | Farbton nach Färben | Farbmeßwerte | | | Entfärbe-% |
|---|---|---|---|---|---|---|
| | | | L | a | b | |
| 1.12 | 1,4-Diamino-2-methylbenzol sulfat: 1,10 g | blauschwarz | unbehandelte Haare: 31,9; | +7,0; | +11,7 | |
| | 1,4-Diamino-2-(2-hydroxyethyl)-benzol sulfat: 1,25 g | | | | | |
| | 5-Amino-2-methyl-phenol: 0,25 g | | Nach dem Färben: 20,4; | +0,8; | -1,3 | |
| | 1,3-Di(2,4-diaminophenoxy)-propan 1,74 g | | nach 1x Entfärben mit b6: 33,5; | +9,0; | +13,2 | 84 |

Tabelle 1 (Fortsetzung)

| Nr. | Entwickler/Kuppler-Kombination | Farbton nach Färben | Farbmeßwerte | | | Entfärbe-% |
|---|---|---|---|---|---|---|
| | | | L | a | b | |
| 1.13 | 1,4-Diamino-2-methylbenzol sulfat: 1,10 g | blauschwarz | unbehandelte Haare: 32,1; +6,7; +11,2 | | | |
| | 1,4-Diamino-2-(2-hydroxyethyl)-benzol sulfat: 1,25 g | | Nach dem Färben: | | | |
| | 5-Amino-2-methyl-phenol: 0,25 g | | 18,9; +0,6; -1,1 | | | |
| | 1,3-Di(2,4-diaminophenoxy)-propan 1,74 g | | nach 1x Entfärben mit b7: 33,9; +8,0; +11,1 | | | 89 |

## Tabelle 1 (Fortsetzung)

| Nr. | Entwickler/Kuppler-Kombination | Farbton nach Färben | Farbmeßwerte | | | Entfärbe-% |
|-----|-------------------------------|---------------------|---|---|---|-----------|
| | | | L | a | b | |
| **1.14** | 1,4-Diamino-2-methylbenzol sulfat: 1,10 g | blauschwarz | unbehandelte Haare: 30,6; +6,8; +10,9 | | | |
| | 1,4-Diamino-2-(2-hydroxyethyl)-benzol sulfat: 1,25 g | | Nach dem Färben: | | | |
| | 5-Amino-2-methyl-phenol: 0,25 g | | 19,5; +0,8; -1,0 | | | |
| | 1,3-Di(2,4-diaminophenoxy)-propan 1,74 g | | nach 1x Entfärben mit **b8**: 32,0; +8,9; +12,8 | | | 82 |

EP 0 943 316 B1

**Beispiele 2.1 bis 2.7;**

Die Färbung erfolgt auf gebleichten Haaren in der in Beispiel 1 angegeben Weise (Konzentration der Farbstoffvorstufen: 0,05 mol/l).

Die Entfärbung erfolgt mit den Entfärbegelen b1 oder b2

Auf das gefärbte Haar trägt man die oben beschriebenen Entfärbemittel auf und läßt jeweils 20 bzw. 30 Minuten bei 37 Grad Celsius unter einer Plastikabdeckung einwirken, danach wird gründlich mit Wasser und einem Shampoo gewaschen, mit Wasser gespült und sodann getrocknet.

Die Ergebnisse der Färbe- und Entfärbebehandlungen sind in der nachfolgenden Tabelle 2 zusammengefaßt.

## Tabelle 2: Färbe- und Entfärbe-Resultate

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.1 | 1,4-Diamino-2-methyl-benzol-sulfat; 1,3-Dihydroxybenzol | braun | b1 | 30 | beige |
| 2.2 | 1,4-Diamino-2-methylbenzolsulfat; 1-Naphthol | dunkel-blau | b2 | 30 | grau |
| 2.3 | 1,4-Diamino-2-methyl-benzol-sulfat; 3-Amino-6-methoxy-2-(methylamino)-pyridin | dunkel-blau | b1 | 30 | bräunlich |

EP 0 943 316 B1

## Tabelle 2 (Fortsetzung)

| Nr. | Entwickler/Kuppler-Kombination | Farbton nach der Färbung | Entfärbe- mittel | Entfärbe- dauer (Minuten) | Farbton nach der Entfärbung |
|---|---|---|---|---|---|
| 2.4 | 4,5-Diamino-1-(2-hydroxyethyl)-1H-pyrazol; 1-Naphthol | intensiv fuchsia | b1 | 30 | rosa |
| 2.5 | 1,4-Diamino-2-(2-hydroxyethyl)-benzol-sulfat; 5-Amino-2-methyl-phenol | intensiv violett | b2 | 20 | schwach gelblich |

EP 0 943 316 B1

## Tabelle 2 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|-----|-------------------------------|-------------------------|-----------------|--------------------------|----------------------------|
| 2.6 | 4,5-Diamino-1-(2-hydroxyethyl)-1H-pyrazol; 2-Amino-4-[(2-hydroxyethyl)amino]-anisol-sulfat | bordeaux-rot | b2 | 30 | schwach bordaux-rot |
| 2.7 | 4-Amino-3-methyl-phenol (0,06%); 1-Naphthol (0,04%); 5-Amino-2-methyl-phenol (0,03%) | rosarot | b2 | 20 | farblos |

**Beispiele 2.8 bis 2.33:**

Die Färbung erfolgt auf gebleichten Haaren in der in Beispiel 1 angegeben Weise (wenn nicht anders angegeben, beträgt die Konzentration der Farbstoffvorstufen 0,05 mol/l).

Die Entfärbung erfolgt mit dem Entfärbegel **b9.1.**

**b9.1. Entfärbeget:**

| | |
|---|---|
| Natriumascorbat | 5,60 g |
| Methylhydroxyethylcellulose (Tylose MHB 10.000P der Firma Hoechst/BRD) | 1,50 g |
| Cystein-Hydrochlorid | 2,50 g |
| Citronensäure | 5,00 g |
| Wasser | ad 100,00 g |

Auf das gefärbte Haar trägt man die oben beschriebenen Entfärbemittel auf und läßt jeweils 20 bzw. 30 Minuten bei 37 Grad Celsius unter einer Plastikabdeckung einwirken, danach wird gründlich mit Wasser und einem Shampoo gewaschen, mit dem Glyoxylsäure-haltigen Nachbehandlungsmittel **b9.2** behandelt, mit Wasser gespült und sodann getrocknet.

**b9.2. Nachbehandlungsmittel**

| | |
|---|---|
| Cetrimoniumchlorid (50 %ig) | 1,00 g |
| Glyoxylsäure (50 %ig) | 1,00 g |
| Hydroxyetheylcellulose | 1,50 g |
| Trinatrium Citrat Dihydrat | 0,30 g |
| Wasser | ad 100,00 g |

Die Ergebnisse der Färbe- und Entfärbebehandlungen sind in der nachfolgenden Tabelle 2a zusammengefaßt.

## Tabelle 2a: Färbe- und Entfärbe-Resultate

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.8 | 4-Amino-3-methylphenol; 2-Amino-4-[(2-hydroxyethylamino]-anisol-sulfat | hell-violett | b9.1 und b9.2 | 30 | schwach gelblich |
| 2.9 | 1,4-Diamino-2-methyl-benzol-sulfat; 2-Amino-4-[(2-hydroxyethyl)amino]-anisol-sulfat | dunkel-blau | b9.1 und b9.2 | 30 | schwach beige |
| 2.10 | 1,4-Diamino-2-methyl-benzol-sulfat; 3-Amino-phenol | intensiv grau-violett | b9.1 und b9.2 | 30 | schwach rötlichbraun |

EP 0 943 316 B1

## Tabelle 2a (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.11 | 1,4-Diamino-2-methyl-benzol-sulfat; 5-[(2-Hydroxyethylamino]-1,3-benzodioxol-hydrochlorid | grün-schwarz | b9.1 und b9.2 | 30 | grünlich |
| 2.12 | 1,4-Diamino-2-methyl-benzol-sulfat; 1,3-Dihydroxy-2-methyl-benzol | braun | b9.1 und b9.2 | 30 | hell braun |
| 2.13 | 4-Amino-3-methyl-phenol; 5-Amino-2-methyl-phenol | lachs-farben | b9.1 und b9.2 | 20 | farblos |

EP 0 943 316 B1

## Tabelle 2a (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.14 | 1,4-Diamino-2-methyl-benzol-sulfat; 1,3-Di(2,4-diaminophenoxy)propan | tiefblau | b9.1 und b9.2 | 30 | schwach orange-beige |
| 2.15 | 4,5-Diamino-1-(2-hydroxyethyl)-1H-pyrazol; 1,3-Dihydroxy-benzol | fuchsia | b9.1 und b9.2 | 30 | schwach rosé |
| 2.16 | 4,5-Diamino-1-(2-hydroxyethyl)-1H-pyrazol; 3-Amino-phenol | rot | b9.1 und b9.2 | 20 | farblos |

## Tabelle 2a (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.17 | 4,5-Diamino-1-(2-hydroxyethyl)-1H-pyrazol; 3-Amino-6-methoxy-2-methylamino-pyridin | blau-schwarz | b9.1 und b9.2 | 30 | beige-grau |
| 2.18 | 4,5-Diamino-1-(2-hydroxyethyl)-1H-pyrazol; 1,3-Dihydroxy-2-methyl-benzol | rot | b9.1 und b9.2 | 30 | schwach rosé |
| 2.19 | 4-Aminophenol; 5-Amino-2-methyl-phenol | lachs-farben | b9.1 und b9.2 | 20 | farblos |

## Tabelle 2a (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.20 | 1,4-Diaminobenzol; 5-Amino-2-methyl-phenol | violett | b9.1 und b9.2 | 30 | schwach beige |
| 2.21 | 2,4,5,6-Tetraaminopyrimidin-sulfat; 5-Amino-2-methyl-phenol | blau | b9.1 und b9.2 | 20 | farblos |
| 2.22 | 2,5-Diamino-4-methyl-phenol-dihydrochlorid; 5-Amino-2-methyl-phenol | tiefblau | b9.1 und b9.2 | 30 | schwach grau |

EP 0 943 316 B1

## Tabelle 2a (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.23 | 4-Amino-3-methylphenol; 2,4-Diamino-6-methylphenol | beige | b9.1 und b9.2 | 30 | schwach gelblich |
| 2.24 | 1,4-Diamino-2-methylbenzolsulfat; 3-Amino-2-methylphenol | braun | b9.1 und b9.2 | 30 | hellbraun |
| 2.25 | 4,5-Diamino-1-(2-hydroxyethyl)-1H-pyrazol; 3-(2-Hydroxethyl)amino-phenol | intensiv rot | b9.1 und b9.2 | 30 | schwach rosé |

EP 0 943 316 B1

## Tabelle 2a (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.26 | 4,5-Diamino-1-(2-hydroxyethyl)-1H-pyrazol; 5-(2-Hydroxyethyl)-amino-2-methylphenol | intensiv orange | b9.1 und b9.2 | 30 | schwach orange |
| 2.27 | 4-Amino-3-methyl-phenol; 5-Amino-2-ethyl-phenol | rosa-orange | b9.1 und b9.2 | 20 | farblos |
| 2.28 | 4,5-Diamino-1-(2-hydroxyethyl)-1H-pyrazol; 2-Methyl-1-naphthol-acetat | intensiv rosa | b9.1 und b9.2 | 30 | schwach rosa |

EP 0 943 316 B1

## Tabelle 2a (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 2.29 | 1,4-Diaminobenzol; 1,3-Diaminobenzol | tiefblau | b9.1 und b9.2 | 30 | beige |
| 2.30 | 1,4-Diamino-2-(2-hydroxyethyl)-benzol-sulfat; 1,3-Diaminobenzol | tiefblau | b9.1 und b9.2 | 20 | schwach gelblich |

EP 0 943 316 B1

## Tabelle 2a (Fortsetzung)

| Nr. | Entwickler/Kuppler-Kombination | Farbton nach dem Färben | Farbmeßwerte | | | Entfärbe-% |
|---|---|---|---|---|---|---|
| | | | L | a | b | |
| 2.31 | 1,4-Diamino-2-methyl-benzol sulfat: 0,44 g | violett | unbehandelte Haare: 37,29; +8,13; +15,88 | | | |
| | 4-Amino-3-methyl phenol: 0,37 g | | | | | |
| | 2,4-Diamino-1-(2-hydroxyethoxy)-benzol dihydrochlorid: 0,48 g | | Nach dem Färben: 26,31; +4,95; +3,55 | | | |
| | 5-Amino-2-methyl-phenol: 0,37 g | | | | | |
| | | | nach 1x Entfärben mit **b9.1 und b9.2:** 39,77; +9,37; +17,90 | | | 79 |

EP 0 943 316 B1

## Tabelle 2a (Fortsetzung)

| Nr. | Entwickler/Kuppler-Kombination | Farbton nach dem Färben | Farbmeßwerte L a b | | | Entfärbe-% |
|---|---|---|---|---|---|---|
| 2.32 | 1,4-Diamino-2-methyl-benzol sulfat: 0,22 g <br> 4-Amino-3-methyl phenol: 0,50 g <br> 5-Amino-2-methyl-phenol: 0,61 g | rot | unbehandelte Haare: <br> 83,29; -0,48; +10,40 <br><br> Nach dem Färben: <br> 47,18; +31,48; +17,32 <br><br> nach 1x Entfärben mit **b9.1 und b9.2:** <br> 80,94; +0,33; +15,74 | | | 90 |

EP 0 943 316 B1

Tabelle 2a (Fortsetzung)

| Nr. | Entwickler/Kuppler-Kombination + Nitrofarbstoffe | Farbton nach Färben | Farbmeßwerte | | | Entfärbe-% |
|---|---|---|---|---|---|---|
| | | | L | a | b | |
| 2.33 | 1,4-Diamino-2-methyl-benzolsulfat: 0,83 g<br>2-Amino-4-[(2-hydroxyethyl)-amino]-anisol-sulfat: 0,42 g<br>4-Amino-3-methylbenzol: 0,46 g<br>2-Amino-6-chlor-4-nitrophenol: 0,225 g | dunkelbraun | unbehandelte Haare:<br>34,41; +7,27; +13,78<br>Nach dem Färben:<br>21,22; +4,66; +3,90<br>nach 1x Entfärben mit **b9.1 und b9.2:**<br>33,87; +7,53; +13,97 | | | 96 |

**Beispiele 2.34 bis 2.36:**

Die Färbung erfolgt auf gebleichten Haaren in der in Beispiel 1 angegeben Weise (wenn nicht anders angegeben, beträgt die Konzentration der Farbstoffvorstufen 0,05 mol/l).

Die Entfärbung erfolgt mit den in den Beispielen **1.1 bis 1.14** beschriebenen Entfärbegelen **b6, b7 oder b8**. Für die Nachbehandlung wird ein Glyoxylsäure enthaltendes Mittel gemäß b9.2 aus Beispiel **2.8** bis **2.33** verwendet.

Auf das gefärbte Haar wird jeweils das oben beschriebene Entfärbemittel **b6, b7 oder b8** aufgetragen. Nach einer Einwirkungszeit von 30 Minuten bei 37 Grad Celsius unter einer Plastikabdeckung wird das Haar gründlich mit Wasser gespült, 5 Minuten lang mit dem Glyoxylsäure enthaltenden Nachbehandlungsmittel **b9.2** behandelt, mit Wasser gespült und sodann getrocknet.

Die Ergebnisse der Färbebehandlungen und Entfärbebehandlungen sind in der nachfolgenden Tabelle 2b zusammengefaßt.

## Tabelle 2b: Färbe- und Entfärberesultate

| Nr. | Entwickler/Kuppler-Kombination | Farbton nach der Färbung | Farbmeßwerte L  a  b | Entfärbe-% |
|---|---|---|---|---|
| 2.34 | 1,4-Diamino-2-methylbenzol sulfat: 1,10 g <br> 1,4-Diamino-2-(2-hydroxyethyl)-benzol sulfat: 1,25 g <br> 5-Amino-2-methyl-phenol: 0,25 g <br> 1,3-Di(2,4-diaminophenoxy)-propan: 1,74 g | blauschwarz | unbehandelte Haare: <br> 31,9; +7,0; +11,7 <br><br> Nach dem Färben: <br> 20,4; +0,8; -1,3 <br><br> nach 1x Entfärben mit **b6** und 5minütiger Nachbehandlung mit **b9.2:** <br> 33,5; +9,0; +13,2 | 84 |

EP 0 943 316 B1

## Tabelle 2b (Fortsetzung)

| Nr. | Entwickler/Kuppler-Kombination | Farbton nach der Färbung | Farbmeßwerte L a b | | | Entfärbe-% |
|---|---|---|---|---|---|---|
| 2.35 | 1,4-Diamino-2-methylbenzol sulfat: 1,10 g<br>1,4-Diamino-2-(2-hydroxyethyl)-benzol sulfat: 1,25 g<br>5-Amino-2-methyl-phenol: 0,25 g<br>1,3-Di(2,4-diaminophenoxy)-propan: 1,74 g | blauschwarz | unbehandelte Haare:<br>32,1; +6,7; +11,2<br><br>Nach dem Färben:<br>18,9; +0,6; -1,1<br><br>nach 1x Entfärben mit **b7** und<br>5minütiger Nachbehandlung mit **b9.2:**<br>33,9; +8,0; +11,1 | | | 89 |

EP 0 943 316 B1

## Tabelle 2b (Fortsetzung)

| Nr. | Entwickler/Kuppler-Kombination | Farbton nach der Färbung | Farbton nach Färben | Farbmeßwerte L | a | b | Entfärbe-% |
|---|---|---|---|---|---|---|---|
| 2.36 | 1,4-Diamino-2-methylbenzol sulfat: 1,10 g | blauschwarz | unbehandelte Haare: 30,6; +6,8; +10,9 | | | | |
| | 1,4-Diamino-2-(2-hydroxyethyl)-benzol sulfat: 1,25 g | | Nach dem Färben: | | | | |
| | 5-Amino-2-methyl-phenol: 0,25 g | | 19,5; +0,8; -1,0 | | | | |
| | 1,3-Di(2,4-diaminophenoxy)-propan: 1,74 g | | nach 1x Entfärben mit **b8** und 5minütiger Nachbehandlung mit **b9.2:** 32,0; +8,9; +12,8 | | | | 82 |

EP 0 943 316 B1

**Beispiele 3.1 bis 3.32:**

Die Färbung erfolgt auf gebleichten Haaren in der in Beispiel 1 angegeben Weise (Konzentration der Farbstoffvorstufen: 0,05 mol/l).

Die Entfärbung erfolgt mit den nachfolgenden Mitteln:

**A: Entfärbegel (entsprechend b4 aus Beispiel 1):**

| | |
|---|---|
| Natriumascorbat | 6,00 g |
| Citronensäure | 6,00 g |
| Hydroxyethylcellulose | 1,50 g |
| Glyoxylsäure | 0,50 g |
| Wasser | ad 100,00 g |

Der pH-Wert des Entfärbegels liegt zwischen 2,5 und 3,5.

**B: Entfärbelösung:**

| | |
|---|---|
| Ascorbinsäure | 10 g |
| Glyoxylsäure | 0,5 g |
| Wasser, vollentsalzt | 90 g |
| | 100 g |

**C: Entfärbebatsam:**

| | |
|---|---|
| Cetylstearylalkohol | 4,50 g |
| Cetyllactat | 0,50 g |
| Dimethicone | 0,50 g |
| Cetyltrimethylammoniumchlorid | 0,65 g |
| Glyoxylsäure | 0,50 g |
| Ascorbinsäure | 6,00 g |
| Wasser, vollentsalzt | ad 100,00 g |

Der pH-Wert des Entfärbebalsams wird mit einer 2 %igen wässrigen NaOH-Lösung auf 2,5 eingestellt.

**D: Entfärbeschaum:**

| | |
|---|---|
| Cetylstearylalkohol | 1,30 g |
| PEG-35 Castor Oil | 0,47 g |
| Cetyltrimethylammoniumchlorid | 0,94 g |
| Ascorbinsäure | 6,00 g |
| Glyoxylsäure | 0,50 g |
| Wasser, vollentsalzt | ad 100,00 g |
| Propan/Butan (5 bar) | 6,00 g |

Der pH-Wert des Entfärbeschaums wird mit Trinatrium Citrat Dihydrat auf 2,5 eingestellt.

Die Haare werden bei 40°C 20 bis 60 Minuten lang mit dem Entfärbemittel behandelt, anschließend gründlich mit Wasser und einem Shampoo gewaschen und sodann getrocknet.

Die Ergebnisse der Färbe- und Entfärbebehandlungen sind in der nachfolgenden Tabelle 3 zusammengefaßt.

**Tabelle 3: Färbe- und Entfärbe-Resultate**

| Nr. | Entwickler/Kuppler-Kombination | Farbton nach der Färbung | Entfärbe- mittel | Entfärbe- dauer (Minuten) | Farbton nach der Entfärbung |
|---|---|---|---|---|---|
| 3.1 | 4-Amino-3-methylphenol; 2-Amino-4-(2-hydroxyethylamino-anisol-sulfat | hell-violett | B | 60 | schwach gelblich |
| 3.2 | 1,4-Diamino-2-methylbenzolfulfat; 2-Amino-4-(2-hydroxyethyl)amino-anisol-sulfat | dunkel-blau | B | 60 | schwach graublau |

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.3 | 1,4-Diamino-2-methylbenzolsulfat; 1,3-Dihydroxybenzol | braun | C | 45 | hell-braun |
| 3.4 | 1,4-Diamino-2-methylbenzolsulfat; 3-Aminophenol | intensiv grau-violett | C | 45 | schwach rötlichbraun |
| 3.5 | 1,4-Diamino-2-methylbenzolsulfat; 1-Naphthol | dunkel-blau | C | 60 | grau |

EP 0 943 316 B1

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.6 | 1,4-Diamino-2-methylbenzolsulfat; 3-Amino-6-methoxy-2-methylamino-pyridin | dunkel-blau | A | 60 | braun |
| 3.7 | 1,4-Diamino-2-methylbenzolsulfat; 5-((2-Hydroxyethylamino)-1,3-benzodioxol-hydrochlorid | grün-schwarz | C | 45 | grünlich |
| 3.8 | 1,4-Diamino-2-methylbenzolsulfat; 1,3-Dihydroxy-2-methylbenzol | braun | C | 60 | hell braun |

EP 0 943 316 B1

Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.9 | 4-Amino-3-methylphenol; 5-Amino-2-methylphenol | lachs-farben | D | 20 | farblos |
| 3.10 | 4,5-Diamino-1-(2-hydroxyethyl)-1H-pyrazol; 1-Naphthol | intensiv fuchsia | B | 45 | rosa |
| 3.11 | 4,5-Diamino-1-(2-hydroxyethyl)-1H-pyrazol; 3,5-Hydroxy-4-methoxy-benzoesäure | rosa-orange | B | 60 | schwach rosa |

EP 0 943 316 B1

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.12 | 1,4-Diamino-2-(2-hydroxyethyl)-benzol-sulfat; 5-Amino-2-methylphenol | intensiv violett | A | 20 | schwach gelblich |
| 3.13 | 4,5-Diamino-1-(2-hydroxyethyl)-1H-pyrazol; 1,3-Dihydroxybenzol | fuchsia | A | 30 | schwach rosé |
| 3.14 | 4,5-Diamino-1-(2-hydroxyethyl)-1H-pyrazol; 3-Aminophenol | rot | A | 20 | schwach beige |

EP 0 943 316 B1

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.15 | 4,5-Diamino-1-(2-hydroxyethyl)-1H-pyrazol; 3-Amino-6-methoxy-2-methylamino-pyridin | blau-schwarz | B | 60 | grau |
| 3.16 | 4,5-Diamino-1-(2-hydroxyethyl)-1H-pyrazol; 2-Amino-4-(2-hydroxyethyl)amino-anisolsulfat | bordeaux-rot | B | 60 | schwach bordaux-rot |

EP 0 943 316 B1

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.17 | 4,5-Diamino-1-(2-hydroxyethyl)-1H-pyrazol; 1,3-Dihydroxy-2-methylbenzol | rot | B | 60 | schwach rosé |
| 3.18 | 4-Aminophenol; 5-Amino-2-methylphenol | lachs-farben | A | 20 | farblos |
| 3.19 | 1,4-Diaminobenzol; 5-Amino-2-methylphenol | violett | A | 45 | schwach beige |

EP 0 943 316 B1

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.20 | 2,4,5,6-Tetraaminopyrimidin-sulfat; 5-Amino-2-methylphenol | blau | A | 20 | farblos |
| 3.21 | 2,5-Diamino-4-methylphenol-dihydrochlorid; 5-Amino-2-methylphenol | tiefblau | A | 60 | schwach grau |
| 3.22 | 1,4-Diamino-2-hydroxymethyl-benzol; 5-Amino-2-methylphenol | beige | A | 60 | schwach orange |

EP 0 943 316 B1

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.23 | 4-Amino-3-methylphenol; 2,4-Diamino-6-methylphenol | beige | A | 60 | schwach gelblich |
| 3.24 | 1,4-Diamino-2-methylbenzolsulfat; 3-Amino-2-methylphenol | braun | A | 60 | hellbraun |
| 3.25 | 4,5-Diamino-1-(2-hydroxyethyl)-1H-pyrazol; 3-(2-Hydroxethyl)amino-phenol | intensiv rot | B | 60 | schwach rosé |

EP 0 943 316 B1

Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.26 | 4,5-Diamino-1-(2-hydroxyethyl)-1H-pyrazol; 5-(2-Hydroxyethyl)amino-2-methylphenol | intensiv oragne | B | 60 | schwach orange |
| 3.27 | 4-Amino-3-methylphenol/ 5-Amino-2-ethylphenol | rosa-orange | A | 20 | farblos |

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.28 | 4,5-Diamino-1-(2-hydroxyethyl)-1H-pyrazol; 2-Methyl-1-naphtholacetat | intensiv rosa | B | 60 | schwach rosa |
| 3.29 | 1,4-Diaminobenzol/ 1,3-Diaminobenzol | tiefblau | A | 60 | beige |
| 3.30 | 1,4-Diamino-2-(2-hydroxyethyl)-benzolsulfat/1,3-Diaminobenzol | tiefblau | A | 20 | schwach gelblich |

Beispiel 4:

Enzymatisch oxidierte Haarfarbe (a)

## Tabelle 3 (Fortsetzung)

| Nr. | Entwickler/Kuppler-Kombination | Farbton nach der Färbung | Entfärbe-mittel | Entfärbe-dauer (Minuten) | Farbton nach der Entfärbung |
|---|---|---|---|---|---|
| 3.31 | 4-Amino-3-methylphenol (0,06%); 1-Naphthol (0,04%); 5-Amino-2-m ethylphenol (0,03%) | rosarot | A | 20 | farblos |

EP 0 943 316 B1

| | |
|---|---|
| Stearylalkohol-polyglykolether (=Steareth 20) | 1,40 g |
| Natriumsulfit | 0,10 g |
| *di*-Natrium-Ethylendiaminotetraacetat | 0,30 g |
| D-Glucose | 1,00 g |
| Glycerin | 1,00 g |
| Isopropanol | 5,00 g |
| 1,2-Propandiol | 2,00 g |
| 1,4-Diamino-2-methylbenzolsulfat | 0,025 M |
| 5-Amino-2-methylphenol | 0,025 M |
| Glucose-Oxidase (EC 1.1.3.4) | 400 units |
| Peroxidase (EC 1.11.1.7) | 400 units |
| 0.1 M Boratpuffer (pH 8,5) | ad 100,00 g |

**Entfärbegel (entsprechend b9.1)**

| | |
|---|---|
| Natriumascorbat | 5,60 g |
| Methylhydroxyethylcellulose (Tylose MHB 10.OOOP der Firma Hoechst/BRD) | 1,50 g |
| Cystein-Hydrochlorid | 2,50 g |
| Citronensäure | 5,00 g |
| Wasser | ad 100,00 g |

**Nachbehandlungsmittel (entsprechend b9.2)**

| | |
|---|---|
| Cetrimoniumchlorid (50 %ig) | 1,00 g |
| Glyoxylsäure (50 %ig) | 1,00 g |
| Hydroxyethylcellulose | 1,50 g |
| Trinatrium Citrat Dihydrat | 0,30 g |
| Wasser | ad 100,00 g |

Das vorstehend beschriebene Haarfärbemittel **(a)** wird auf gebleichtes Haar aufgetragen. Nach einer Einwirkzeit von 60 Minuten bei Raumtemperatur (25 Grad Celsius) werden die Haare gewaschen und getrocknet.

Die tiefviolett gefärbten Haare werden anschließend 20 Minuten bei 40°C mit dem Entfärbegel **b9.1** und 2 bis 3 Minuten mit dem Nachbehandlungsmittel **b9.2** behandelt.

Die Haare werden sodann gründlich gewaschen und getrocknet. Das Haar erhält annähemd seine Ausgangsfarbe zurück.

**Beispiel 5: Zweikomponenten-Entfärbeemulsion**

| | | |
|---|---|---|
| Komponente 1: | Cetylstearylalkohol | 4,50 g |
| | Cetyllactat | 0,50 g |
| | Dimethicone | 0,50 g |
| | Cetyltrimethylammoniumchlorid | 0,65 g |
| | Glyoxylsäure | 0,50 g |
| | Wasser, vollentsalzt | ad 92,00 g |
| Komponente 2: | L-Cystein | 2,00 g |
| | Ascorbinsäure (pulverförmig) | 6,00 g |

Tabelle fortgesetzt

100,00 g

Die Komponente 1 wird unmittelbar vor der Anwendung mit der Komponente 2 vermischt und der pH-Wert der so erhaltenen gebrauchsfertigen Entfärbezubereitung wird mit Trinatrium Citrat Dihydrat auf 2,5 eingestellt.

**Beispiel 6: Entfärbegel**

| | |
|---|---|
| 8,00 g | Ascorbinsäure |
| 2,00 g | L-Cystein |
| 0,08 g | a-Ketoglutarsäure |
| 2,00 g | Hydroxyethylcellulose |
| 0,50 g | Silica |

Der pH-Wert wird mit Trinatrium Citrat Dihydrat auf 2,5 bis 3,0 eingestellt. Die Mischung wird vor der Anwendung mit 89,34 g warmem Wasser versetzt und gut vermischt. Das so erhaltene Entfärbegel kann zur Entfärbung von Fasern oder Haaren, welche mit oxidativen Farben gefärbt sind, verwandt werden.

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Der Wert D gibt die Farbdifferenz an, die zwischen den unbehandelten und den gefärbten bzw. entfärbten Strähnchen besteht. Er wird folgendermaßen bestimmt:

$$D = \sqrt{(L_i - L_0)^2 + (a_i - a_0)^2 + (b_i - b_o)^2}$$

wobei $L_0$, $a_0$ und $b_0$ die Farbmesswerte von unbehandeltem Haar und $L_i$, $a_i$ und $b_i$ die Werte des behandelten Haares darstellen. Die Entfärberate in Prozent wurde folgendermaßen ermittelt:

$$\text{Entfärbe-\%} = [1 - (D \text{ nach Entfärbung} / D \text{ nach Färbung})] \times 100.$$

Alle in der vorliegenden Anmeldung genannten Prozentangaben stellen soweit nicht anders angegeben Gewichtsprozente dar.

**Patentansprüche**

1. Mittel zur reduzierenden Entfärbung von mit Oxidationsfarbstoffen und/oder direktziehenden Farbstoffen gefärbten Fasern, **dadurch gekennzeichnet, daß** es eine Kombination aus a) mindestens einem Redukton und/oder mindestens einem Thiol und/oder mindestens einem Sulfit und b) mindestens einer $\alpha$-Oxocarbonsäure oder derem physiologisch verträglichen Salz enthält.

2. Mittel nach Anspruch 1, **dadurch gekennzeichnet, daß** es eine Kombination aus a) mindestens einem Redukton und mindestens einem Thiol und mindestens einem Sulfit und b) mindestens einer a-Oxocarbonsäure oder derem physiologisch verträglichen Salz enthält.

3. Mittel nach Anspruch 1 oder 2, **dadurch gekennzeichnet, daß** das Redukton ausgewählt ist aus Ascorbinsäure, Isoascorbinsäure oder deren Salzen und Estern, Hydroxypropandial, 2,3-Dihydroxy-2-cyclopenten-1-on und Mischungen dieser Verbindungen.

4. Mittel nach Anspruch 3, **dadurch gekennzeichnet, daß** die Ascorbinsäure oder Isoascorbinsäure in situ aus Alkalimetallascorbaten, Erdalkalimetallascorbaten, Alkalimetallisoascorbaten und Erdalkalimetallisoascorbaten und einer Säure erzeugt wird.

5. Mittel nach Anspruch 1 oder 2, **dadurch gekennzeichnet, daß** das Thiol ausgewählt ist aus Cystein oder dessen Salzen, N-Acetylcystein, Cysteamin oder dessen Salzen, Mercaptoacetaldehyd, Penicillamin, Glutathion, Homocystein oder dessen Salzen und Calciumthiolglykolat.

6. Mittel nach Anspruch 1 oder 2, **dadurch gekennzeichnet, daß** das Sulfit ausgewählt ist aus Alkalisulfiten und Erdalkalisulfiten.

7. Mittel nach Anspruch 1 oder 2, **dadurch gekennzeichnet, daß** die $\alpha$-Oxocarbonsäure ausgewählt ist aus Glyoxylsäure, $\alpha$-Ketoglutarsäure, Oxalessigsäure, Brenztraubensäure sowie den Alkalisalzen oder Erdalkalisalzen dieser Verbindungen.

8. Mittel nach einem der Ansprüche 1 bis 7, **dadurch gekennzeichnet, daß** das Redukton in einer Menge von 1 bis 50 Gewichtsprozent enthalten ist.

9. Mittel nach einem der Ansprüche 1 bis 8, **dadurch gekennzeichnet, daß** das Thiol in einer Menge von 0,1 bis 10 Gewichtsprozent

10. Mittel nach einem der Ansprüche 1 bis 9, **dadurch gekennzeichnet, daß** das Sulfit in einer Menge von 0,001 bis 5 Gewichtsprozent enthalten ist.

11. Mittel nach einem der Ansprüche 1 bis 10, **dadurch gekennzeichnet, daß** die $\alpha$-Oxocarbonsäure oder deren physiologisch verträgliches Salz in einer Menge von 0,1 bis 10 Gewichtsprozent enthalten ist.

12. Mittel nach einem der Ansprüche 1 bis 11, **dadurch gekennzeichnet, daß** es als Lösung, Emulsion, Schaum, Creme, Gel, Pulver, Granulat oder als Brausetablette vorliegt.

13. Mittel nach einem der Ansprüche 1 bis 12, **dadurch gekennzeichnet, daß** es einen pH-Wert von 1,8 bis 6 aufweist.

14. Mehrkomponenten-Kit zur Färbung und Entfärbung von Fasern, insbesondere von Haaren, **dadurch gekennzeichnet, daß** er als Komponente (I) ein Mittel zur oxidativen oder nicht-oxidativen Färbung von Fasern, und als Komponente (II) ein Mittel zur reduzierenden Entfernung der Färbung nach einem der Ansprüche 1 bis 13 enthält.

15. Mehrkomponenten-Kit nach Anspruch 14, **dadurch gekennzeichnet, daß** die Komponente (I) eine Farbträgermasse auf der Basis von Farbstoffvorstufen, die bei Zugabe eines Oxidationsmittels einen Oxidationsfarbstoff bilden, enthält.

16. Mehrkomponenten-Kit nach 15, **dadurch gekennzeichnet, daß** die Komponente (I) zusätzlich mindestens einen direktziehenden Farbstoff enthält.

17. Mehrkomponenten-Kit nach Anspruch 14, **dadurch gekennzeichnet, daß** die Komponente (I) ein nicht-oxidatives Färbemittel auf der Basis von direktziehenden Farbstoffen ist.

18. Verfahren zur reduzierenden Entfärbung von mit einer Kombination aus Oxidationsfarbstoffen und/oder direktziehenden Farbstoffen gefärbten Fasern, **dadurch gekennzeichnet, daß** man eine Zubereitung nach einem der Ansprüche 1 bis 13, für eine Dauer von 5 bis 60 Minuten bei einer Temperatur von 20 bis 50 °C auf die Fasern einwirken läßt.

19. Verfahren zur reduzierenden Entfärbung von mit einer Kombination aus Oxidationsfarbstoffen und/oder direktziehenden Farbstoffen gefärbten Fasern, **dadurch gekennzeichnet, daß** man eine mindestens ein Redukton und/oder mindestens ein Thiol und/oder mindestens ein Sulfit enthaltende Zubereitung für eine Dauer von 5 bis 60 Minuten bei einer Temperatur von 20 bis 50 °C auf die Fasern einwirken läßt und anschließend die Fasern mit einer $\alpha$-Oxocarbonsäuren-haltigen Spülung behandelt.

**20.** Verfahren nach Anspruch 18, **dadurch gekennzeichnet, daß** man die Fasern im Anschluß an die Entfärbung mit einem Mittel nach einem der Ansprüche 1 bis 13 mit einer α-Oxocarbonsäuren-haltigen Spülung behandelt.

**Claims**

**1.** Agent for the reductive removal of colour from fibres, in particular hair, dyed with oxidation dyes and/or direct dyes, **characterized in that** it comprises a combination of a) at least one reductone and/or at least one thiol and/or at least one sulphite and b) at least one α-oxocarboxylic acid or the physiologically compatible salt thereof.

**2.** Agent according to Claim 1, **characterized in that** it comprises a combination of a) at least one reductone and at least one thiol and at least one sulphite and b) at least one α-oxocarboxylic acid or the physiologically compatible salt thereof.

**3.** Agent according to Claim 1 or 2, **characterized in that** the reductone is chosen from ascorbic acid, isoascorbic acid or salts and esters thereof, hydroxypropanedial, 2,3-dihydroxy-2-cyclopenten-1-one and mixtures of these compounds.

**4.** Agent according to Claim 3, **characterized in that** the ascorbic acid or isoascorbic acid is generated in situ from alkali metal ascorbates, alkaline earth metal ascorbates, alkali metal isoascorbates and alkaline earth metal isoascorbates and an acid.

**5.** Agent according to Claim 1 or 2, **characterized in that** the thiol is chosen from cysteine or salts thereof, N-acetylcysteine, cysteamine or salts thereof, mercaptoacetaldehyde, penicillamine, glutathione, homocysteine or salts thereof and calcium thioglycolate.

**6.** Agent according to Claim 1 or 2, **characterized in that** the sulphite is chosen from alkali metal sulphites and alkaline earth metal sulphites.

**7.** Agent according to Claim 1 or 2, **characterized in that** the α-oxocarboxylic acid is chosen from glyoxylic acid, α-ketoglutaric acid, oxalacetic acid, pyruvic acid, and the alkali salts or alkaline earth metal salts of these compounds.

**8.** Agent according to one of Claims 1 to 7, **characterized in that** the reductone is present in an amount of from 1 to 50 per cent by weight.

**9.** Agent according to one of Claims 1 to 8, **characterized in that** the thiol is present in an amount of from 0.1 to 10 per cent by weight.

**10.** Agent according to one of Claims 1 to 9, **characterized in that** the sulphite is present in an amount of from 0.001 to 5 per cent by weight.

**11.** Agent according to one of Claims 1 to 10, **characterized in that** the α-oxocarboxylic acid or physiologically compatible salt thereof is present in an amount of from 0.1 to 10 per cent by weight.

**12.** Agent according to one of Claims 1 to 11, **characterized in that** it is in the form of a solution, emulsion, foam, cream, gel, powder, granulate or effervescent tablet.

**13.** Agent according to one of Claims 1 to 12, **characterized in that** it has a pH of from 1.8 to 6.

**14.** Multicomponent kit for the dyeing of and removal of colour from fibres, in particular hair, **characterized in that** it contains, as component (I), an agent for the oxidative or non-oxidative dyeing of fibres, and, as component (II), an agent for the reductive removal of the colour according to one of Claims 1 to 13.

**15.** Multicomponent kit according to Claim 14, **characterized in that** the component (I) contains a colour carrier mass based on dye precursors which, upon the addition of an oxidizing agent, form an oxidation dye.

**16.** Multicomponent kit according to Claim 15, **characterized in that** the component (I) additionally contains at least one direct dye.

17. Multicomponent kit according to Claim 14, **characterized in that** the component (I) is a non-oxidative colourant based on direct dyes.

18. Method for the reductive removal of colour from fibres dyed with a combination of oxidation dyes and/or direct dyes, **characterized in that** a preparation according to one of Claims 1 to 13 is left to act on the fibres for a period of from 5 to 60 minutes at a temperature of from 20 to 50°C.

19. Method for the reductive removal of colour from fibres dyed with a combination of oxidation dyes and/or direct dyes, **characterized in that** a preparation comprising at least one reductone and/or at least one thiol and/or at least one sulphite is left to act on the fibres for a period of from 5 to 60 minutes at a temperature of from 20 to 50°C and then the fibres are treated with a rinse containing $\alpha$-oxocarboxylic acids.

20. Method according to Claim 18, **characterized in that**, following colour removal with an agent according to one of Claims 1 to 13, the fibres are treated with a rinse containing $\alpha$-oxocarboxylic acids.

**Revendications**

1. Composition pour la décoloration réductrice de fibres colorées avec des colorants d'oxydation et/ou des colorants directs, **caractérisée en ce qu'**elle contient une association de a) au moins une réductone et/ou au moins un thiol et/ou au moins un sulfite et b) au moins un acide $\alpha$-oxocarboxylique ou un sel physiologiquement acceptable d'un tel acide.

2. Composition selon la revendication 1, **caractérisée en ce qu'**elle contient une association de a) au moins une réductone et au moins un thiol et au moins un sulfite et b) au moins un acide $\alpha$-oxocarboxylique ou un sel physiologiquement acceptable d'un tel acide.

3. Composition selon la revendication 1 ou 2,
**caractérisée en ce que** la réductone est choisie parmi l'acide ascorbique, l'acide isoascorbique ou leurs sels et esters, l'hydroxypropanedial, la 2,3-dihydroxy-2-cyclopentén-1-one et des mélanges de ces composés.

4. Composition selon la revendication 3, **caractérisée en ce que** l'acide ascorbique ou l'acide isoascorbique est formé in situ à partir d'ascorbates de métaux alcalins, d'ascorbates de métaux alcalino-terreux, d'isoascorbates de métaux alcalins et d'isoascorbates de métaux alcalino-terreux et d'un acide.

5. Composition selon la revendication 1 ou 2,
**caractérisée en ce que** le thiol est choisi parmi la cystéine ou ses sels, la N-acétylcystéine, la cystéamine ou ses sels, le mercaptoacétaldéhyde, la pénicillamine, le glutathion, l'homocystéine ou ses sels et le thioglycolate de calcium.

6. Composition selon la revendication 1 ou 2,
**caractérisée en ce que** le sulfite est choisi parmi des sulfites alcalins et des sulfites alcalino-terreux.

7. Composition selon la revendication 1 ou 2,
**caractérisée en ce que** l'acide $\alpha$-oxocarboxylique est choisi parmi l'acide glyoxylique, l'acide $\alpha$-cétoglutarique, l'acide oxaloacétique, l'acide pyruvique ainsi que les sels alcalins ou les sels alcalino-terreux de ces composés.

8. Composition selon l'une quelconque des revendications 1 à 7, **caractérisée en ce que** la réductone est contenue en une quantité de 1 à 50 % en poids.

9. Composition selon l'une quelconque des revendications 1 à 8, **caractérisée en ce que** le thiol est contenu en une quantité de 0,1 à 10 % en poids.

10. Composition selon l'une quelconque des revendications 1 à 9, **caractérisée en ce que** le sulfite est contenu en une quantité de 0,001 à 5 % en poids.

**11.** Composition selon l'une quelconque des revendications 1 à 10, **caractérisée en ce que** l'acide α-oxocarboxylique ou un sel physiologiquement acceptable de celui-ci est contenu en une quantité de 0,1 à 10 % en poids.

**12.** Composition selon l'une quelconque des revendications 1 à 11, **caractérisée en ce qu'**elle se trouve sous forme de solution, émulsion, mousse, crème, gel, poudre, produit granulé ou sous forme de comprimé effervescent.

**13.** Composition selon l'une quelconque des revendications 1 à 12, **caractérisée en ce qu'**elle présente un pH de 1,8 à 6.

**14.** Nécessaire à plusieurs composants pour la coloration et la décoloration de fibres, en particulier des cheveux, **caractérisé en ce qu'**il contient en tant que composant (I) une composition pour la coloration oxydante ou non oxydante de fibres, et en tant que composant (II) une composition pour l'élimination réductrice de la coloration selon les revendications 1 à 13.

**15.** Nécessaire à plusieurs composants selon la revendication 14, **caractérisé en ce que** le composant (I) contient une matière colorante à base de précurseurs de colorants, qui, lors de l'addition d'un oxydant, forment un colorant d'oxydation.

**16.** Nécessaire à plusieurs composants selon la revendication 15, **caractérisé en ce que** le composant (I) contient en outre au moins un colorant direct.

**17.** Nécessaire à plusieurs composants selon la revendication 14, **caractérisé en ce que** le composant (I) est un agent colorant non oxydant à base de colorants directs.

**18.** Procédé pour la décoloration réductrice de fibres colorées avec une association de colorants d'oxydation et/ou de colorants directs, **caractérisé en ce qu'**on fait agir sur les fibres une préparation selon l'une quelconque des revendications 1 à 13, pendant une durée de 5 à 60 minutes, à une température de 20 à 50 °C.

**19.** Procédé pour la décoloration réductrice de fibres colorées avec une association de colorants d'oxydation et/ou de colorants directs, **caractérisé en ce qu'**on fait agir sur les fibres, pendant une durée de 5 à 60 minutes, à une température de 20 à 50 °C, une préparation contenant au moins une réductone et/ou au moins un thiol et/ou au moins un sulfite, et on traite ensuite les fibres par une solution de rinçage contenant des acides α-oxocarboxyliques.

**20.** Procédé selon la revendication 18, **caractérisé en ce qu'**à la suite de la décoloration par une composition selon l'une quelconque des revendications 1 à 13 on traite les fibres par une solution de rinçage contenant des acides α-oxocarboxyliques.